# EUROPÄISCHE PATENTSCHRIFT

(11) **EP 0 446 759 B1**
(45) Veröffentlichungstag und Bekanntmachung des Hinweises auf die Patenterteilung: **27.07.1994**
(21) Anmeldenummer: 91103277.9
(22) Anmeldetag: 05.03.1991
(51) Int. Cl.: C07C 49/597, C07C 45/54

(54) **Verfahren zur Herstellung von Cyclopentenonen**
Process for the production of cyclopentenones
Procédé pour la préparation de cyclopenténones

(30) Priorität: 13.03.1990 DE 4007925
(43) Veröffentlichungstag der Anmeldung: 18.09.1991
(73) Patentinhaber: BASF Aktiengesellschaft, 67063 Ludwigshafen (DE)
(72) Erfinder: Merger, Franz, Dr., W-6710 Frankenthal (DE); Witzel, Tom, Dr., W-6700 Ludwigshafen (DE); Horler, Hans, Dr., W-6102 Pfungstadt (DE); Lermer, Helmut, Dr., W-6700 Ludwigshafen (DE)

(56) Entgegenhaltungen:
- EP-A- 0 297 447
- DE-A- 3 622 012
- DE-B- 2 439 742
- PATENT ABSTRACTS OF JAPAN, unexamined applications, Sektion C, Band 4, Nr. 12, 29, Januar 1980; THE PATENT OFFICE JAPANESE GOVERNMENT, Seite 143 C 71

## Beschreibung

Die vorliegende Erfindung betrifft ein Verfahren zur Herstellung von 2-Cyclopentenonen durch Umsetzung von Pentensäureestern an aciden Heterogenkatalysatoren.

Bisher bekannte Syntheseverfahren für Cyclopentenon gehen zumeist bereits vom cyclischen Kohlenstoffgerüst aus, z.B.:
- aus Cyclopentadien und Persäure über Epoxycyclopenten (Org. Synth. Coll. V, S. 326 - 328, S. 414 - 418; Yukagaku, (1980) 29, S. 920 - 925)
- durch Oxidation von Cyclopenten entweder elektrochemisch (Chem. Pharm. Bull. (1985) 33, S. 4798 - 4802) oder durch Peroxide (z.B. JP 59/80 619)
- durch Dehydrierung von Cyclopentanon (z.B. an Übergangsmetallkatalysatoren, DE-A-20 50 565).

Ausgehend von offenkettigen Edukten ist z.B. die Umsetzung von 3-Hexen-1,6-disäureestern mit Hilfe stöchiometrischer Mengen starker Basen, wie Natriumalkoholaten, über das Reaktionszwischenprodukt 2-Cyclopenten-2-carbonester in zwei Stufen zu 2-Cyclopentenon beschrieben (Dieckmann-Kondensation, z.B. JP 77/118 447). Hierbei sind drei Verfahrenschritte notwendig (Kondensation, Neutralisation, Verseifung und Decarboxylierung), wobei die Neutralisation zu einem erheblichen Zwangsanfall an Neutralsalzen führt.

Substituierte gamma-Lactone, die z. B. aus ungesättigten Carbonsäureestern zugänglich sind, können mit starken Säuren (Schwefelsäure, Phosphorpentoxid) in die Cyclopentenon-Derivate überführt werden (z.B. Synth. Commun. (1979) 9, S. 545 - 552; EP-A-194 144; JP 58/208 247; JP 75/012 423).

Es ist ebenfalls bekannt, direkt ungesättigte Carbonsäuren durch intramolekulare Acylierung unter Abspaltung von Wasser in Gegenwart von z.B. Schwefelsäure, Schwefelsäure-Essigsäureanhydrid, Zinkchlorid-Essigsäureanhydrid, Trifluoressigsäureanhydrid, Phosphor(V)oxid oder Phosphor(V)chlorid in Cyclopentenon-Derivate zu überführen (Houben-Weyl Bd. 7/2a, (1973) S. 448 - 457; Angew. Chem. (1984) 96, S. 815; Synthesis (1973) S. 397 - 412; JP 54/095 535). JP 54/148 740 beschreibt die Gasphasenumsetzung von 4-Methyl-pentensäuren zum 3-Methyl-2-cyclopenten-1-on an Polyphosphorsäure-, Siliciumdioxid/Aluminiumoxid- oder Borphosphatkontakten. Diese Umsetzungen der freien, ungesättigten Carbonsäuren verlaufen für substituierte Cyclopentenone mit befriedigenden bis guten Ausbeuten. Unsubstituiertes 2-Cyclopentenon jedoch erhält man aus Pentensäure nur in Spuren (J. Chem. Soc. (1957) S. 1435 - 1437, J. Chem. Soc. (1968) 217 - 225).

Aus der EP-A-297 447 ist ein Verfahren zur Herstellung von 2-Cyclopentenonen durch Umsetzung von Hexendisäuren oder Estern an festen, oxidischen Katalysatoren bei Temperaturen von 150 bis 450°C und Drücken bis zu 20 bar bekannt, wobei das Ausgangsmaterial eine Abgangsgruppe enthält.

Der vorliegenden Erfindung lag daher die Aufgabe zugrunde, ein wirtschaftlich attraktives und technisch einfach durchführbares Verfahren zur Herstellung von Cyclopentenonen insbesondere zur Herstellung von unsubstituiertem 2-Cyclopentenon zu finden.

Demgemäß wurde ein neues Verfahren zur Herstellung von Cyclopentenonen der allgemeinen Formel I, 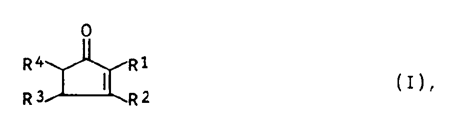
in der R¹ bis R⁴ unabhängig voneinander Wasserstoff oder C₁- bis C₈-Alkyl kedeuten, gefunden, welches dadurch gekennzeichnet ist, daß man Carbonsäureester der allgemeinen Formeln IIa und/oder IIb und/oder IIc

R¹CH=CR²-CHR³-CHR⁴-CO₂R⁵ (IIa)

und/oder

R¹CH₂-CR²=CR³-CHR⁴-CO₂R⁵ (IIb)

und/oder

R¹CH₂-CHR²-CR³=CR⁴-CO₂R⁵ (IIc),

in der R¹ bis R⁴ die obengenannten Bedeutungen haben und R⁵ für C₁- bis C₈-Alkyl steht, an aciden Heterogenkatalysatoren bei Temperaturen von 50 bis 800⁰C und Drücken von 0,001 bis 50 bar umsetzt.

Die Reaktion kann diskontinuierlich oder vorzugsweise kontinuierlich, in der Flüssigphase oder in der Gasphase bei 50 bis 800⁰C und 0,001 bis 50 bar durchgeführt werden.

Die Flüssligphasenreaktion läßt sich bei Temperaturen von 50 bis 200⁰C und Drücken von 0,5 bis 5 bar durchführen.

Die bevorzugte Gasphasenreaktion kann beispielsweise bei Temperaturen von 150 bis 800⁰C und Drücken von 0,001 bis 50 bar, vorzugsweise bei 200 bis 600⁰C und Drücken von 0,1 bis 5 bar, besonders bevorzugt bei 280 bis 500⁰C und Drücken von 0,5 bis 2 bar durchgeführt werden. Bei der Umsetzung in der Gasphase hält man vorteilhaft eine Katalysatorbelastung von 0,01 bis 40, insbesondere von 0,05 bis 10 g Ausgangsstoffe der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Nach der Umsetzung werden die entstandenen Produkte durch übliche Verfahren, z.B. durch Destillation aus dem Reaktionsgemisch isoliert; nicht umgesetzte Ausgangsstoffe werden gegebenenfalls in die Umsetzung zurückgeführt.

Die Verbindungen IIa, IIb und IIc können einzeln oder als Gemische eingesetzt werden sowie gegebenenfalls andere Doppelbindungsisomere.

Die Substituenten R¹ bis R⁴ stehen unabhängig voneinander für Wasserstoff oder einen organischen Rest, wie C₁- bis C₈-Alkyl, z.B. Methyl, Ethyl, n-Propyl, iso-Propyl, n-Butyl, iso-Butyl, sec.-Butyl oder tert.-Butyl bevorzugt für Wasserstoff und Methyl.

Besonders bevorzugt bedeutet R¹ bis R⁴ Wasserstoff.

Der Substituent R⁵ in den Verbindungen II steht für C₁- bis C₈-Alkyl, vorzugsweise C₁- bis C₄-Alkyl, wie Methyl, Ethyl, n-Propyl, iso-Propyl, n-Butyl, iso-Butyl, sec.-Butyl oder tert.-Butyl, besonders bevorzugt für Methyl.

Ausgangsverbindungen der Formeln IIa, IIb und IIc sind beispielsweise:
Pentensäuremethylester
4-Methyl-pentensäuremethylester
3-Methyl-pentensäuremethylester
2-Methyl-pentensäuremethylester
2,4-Dimethyl-pentensäuremethylester
2,3-Dimethyl-pentensäuremethylester
3,4-Dimethyl-pentensäuremethylester
2,2-Dimethyl-pentensäuremethylester
Hexensäuremethylester

Endprodukte der Formel I sind beispielsweise:
2-Cyclopenten-1-on
2-Methyl-2-cyclopenten-1-on
3-Methyl-2-cyclopenten-1-on
4-Methyl-2-cyclopenten-1-on
5-Methyl-2-cyclopenten-1-on
3,5-Dimethyl-2-cyclopenten-1-on
4,5-Dimethyl-2-cyclopenten-1-on
3,4-Dimethyl-2-cyclopenten-1-on
5,5-Dimethyl-2-cyclopenten-1-on

Neben Cyclopentenonen der Formel I erhält man durch Retrocarbonylierung auch Diene der Formel III, sowie deren CC-Doppelbindungsisomere

R¹CH=CR²-CR³=CHR⁴ (III).

Bei Einsatz von ungesättigten Carbonestern mit R¹ = Alkyl ist zusätzlich die Bildung von Cyclohexenon-Derivaten möglich: So reagiert Hexensäuremethylester (R¹=R⁵=CH₃, R²=R³=R⁴=H) nach dem erfindungsgemäßen Verfahren zu einem Gemisch aus 2-Methyl-2-cyclopenten-1-on und 2-Cyclohexen-1-on in variierbarem Verhältnis.

Das erfindungsgemäße Verfahren ist synthetisch interessant insbesondere für die Umwandlung von Pentensäuremethylester-Gemischen in 2-Cyclopentenon (R¹ bis R⁴ = H), denn die entsprechenden Pentensäureester sind durch Carbonylierung von Butadien gut zugänglich.

Als acide Heterogenkatalysatoren eignen sich insbesondere saure Zeolithe, Phosphate oder sauer wirkende Oxide von Elementen der dritten und vierten Hauptgruppe sowie der zweiten bis sechsten Nebengruppe des periodischen Systems (PSE).

Besonders vorteilhaft werden saure zeolithische Katalysatoren eingesetzt.

Zeolithe sind kristalline Aluminiumsilikate, die eine hochgeordnete Struktur mit einem starren dreidimensionalen Netzwerk von SiO₄- und AlO₄-Tetraedern aufweisen, die durch gemeinsame Sauerstoffatome verbunden sind. Das Verhältnis der Si- und Al-Atome zu Sauerstoff beträgt 1 : 2 (siehe Ullmanns Encyclopädie der technischen Chemie, 4. Auflage, Band 24, Seite 575 (1983)). Die Elektrovalenz der Aluminium enthaltenden Tetraeder ist durch Einschluß von Kationen in den Kristall, z. B. eines Alkali- oder Wasserstoffions ausgeglichen. ein Kationenaustausch ist möglich.

In den oben genannten Tetraedern können außer Silicium und Aluminium auch andere Elemente, wie B, Ga, Fe, Cr, V, As, Sb, Be, Ge, Ti, Zr und Hf, eingebaut werden.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z. B. Y-oder X-Zeolithe, oder L-Zeolithe. In diese Gruppe von Zeolithen gehören auch die sogenannten "ultrastabilen" Zeolithe des Faujasittyps, d. h. dealuminierte Zeolithe. Verfahren zur Herstellung solcher Zeolithe sind beschrieben in "Catalysis by Zeolites", Band 5 aus 'Studies in Surface Science and Catalysis' ed. B. Imelik et al. Elsevier Scientific Publishing Comp., 1980, S. 203 und "Crystal Structures of Ultra-stable Faujasites" Advances in Chemistry Series Nr. 101, American Chemical Society, Washington, DC, S. 226 ff (1971) und in US-A-4 512 961.

Besonders vorteilhaft sind Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., C. Aufl., 9d. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische Sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren.

Der Aluminosilikatzeolith wird z. B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Diaminohexan- oder 1,3-Diaminopropan- oder Triethylentetramin-Lösung mit oder insbesondere ohne Alkali- oder Erdalkalizusatz bei 100 bis 220⁰C unter autogenem Druck hergestellt. Auch gehören hierzu die isotaktischen Zeolithe nach EP-A-34 727. Die erhaltenen Aluminosilikatzeolithe enthalten je nach Wahl der Einsatzstoffmengen ein SiO₂/Al₂O₃-Verhältnis von 10 bis 40.000. Auch lassen sich derartige Aluminosilikatzeolithe in etherischem Medium wie Diethylenglykoldimethylether, in alkoholischem Medium wie Methanol bzw. Butan-1,4-diol oder in Wasser synthetisieren.

Der Borosilikatzeolith wird z. B. bei 90 bis 200⁰C unter autogenem Druck synthetisiert, indem man eine Borverbindung z. B. H₃BO₃, mit einer Siliciumverbindung, vorzugsweise hochdispersem Siliciumdioxid in wäßriger Aminlösung, insbesondere in 1,6-Diaminohexan- oder 1,3-Diaminopropan- oder Triethylentetramin-Lösung mit und insbesondere ohne Alkali- oder Erdalkalizusatz zur Reaktion bringt. Auch gehören hierzu die isotaktischen Zeolithe nach EP-A-34 727. Solche Borosilikatzeolithe können ebenfalls hergestellt werden, wenn man die Reaktion statt in wäßriger Aminlösung in etherischer Lösung, z. B. Diethylenglykoldimethylether oder in alkoholischer Lösung, z. B. Hexan-1,6-diol durchführt.

Den Eisensilikatzeolith erhält man z. B. aus einer Eisenverbindung, vorzugsweise Fe₂(SO₄)₃ und einer Siliciumverbindung, vorzugsweise hochdispersem Siliciumdioxid, in wäßriger Aminlösung, insbesondere 1,6-Diaminohexan, mit oder ohne Alkali- oder Erdalkalizusatz bei 100 bis 220⁰C unter autogenem Druck.

Zu den verwendbaren siliciumreichen Zeolithen (SiO₂/Al₂O₃) gehören auch die sogenannten ZSM-Typen, Ferrierit, NU-1 und Silicalite® (ein Molekularsieb nach US-A-4 061 724).

Die so hergestellten Alumino-, Boro- und Eisensilikatzeolithe können nach ihrer Isolierung, Trocknung bei 100 bis 160⁰C, vorzugsweise 110⁰C und Calcinierung bei 450 bis 550⁰C, vorzugsweise 500⁰C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25 : 75 bis 95 : 5, bevorzugt 75 : 25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110⁰C/16 h getrocknet und bei 500⁰C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z. B. Ethylcellulose, Stearinsäure, Kartoffelstärke, Ameisensäure, Oxalsäure, Essigsäure, Salpetersäure, Ammoniak, Amine und Graphit oder deren Gemische verwendet werden.

Liegt der Zeolith aufgrund der Art seiner Herstellung nicht in der katalytisch aktiven, aciden H-Form vor, sondern z. B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen der zeolithischen Katalysatoren eventuell eine durch Koksabscheidung bedingte Desaktivierung eintritt, empfiehlt es sich, die Zeolithe durch Abtrennen der Koksablagerung mit Luft oder mit einem Luft/N₂-Gemisch bei 400 bis 550⁰C, bevorzugt 500⁰C, zu regenerieren. Die Zeolithe erhalten dadurch ihre Anfangsaktivität zuruck.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z. B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Ba, Metalle der 3., 4. und 5. Nebengruppe wie Al, Ga, Sn, Pb, Bi, Übergangsmetalle oder 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Yb und U eingesetzt.

Zweckmäßigerweise führt man die Dotierung so durch, daß man z. B. den verformten Zeolithen in einem Steigrohr vorlegt und bei 20 bis 100⁰C z. B. in eine wäßrige oder ammoniakalische Lösung eines Halogenids oder eines Nitrats der voranbeschriebenen Metalle überleitet. Ein derartiger Ionenaustausch kann z. B. an der Wasserstoff-, Ammonium- und Akaliform des Zeolithen vorgenommen werden. Eine weitere Möglichkeit der Metallaufbringung auf den Zeolithen ist gegeben, indem man das zeolithische Material z. B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z. B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150⁰C getrocknet und bei ca. 550⁰C calciniert. Dieser Tränkvorgang kann mehrmals hintereinander vorgenommen werden, um den gewünschten Metallgehalt einzustellen.

Auch ist es möglich, z. B. eine wäßrige Ni(CO₃)₂-Lösung oder ammoniakalische Pd(NO₃)₂-Lösung herzustellen und darin den reinen pulverförmigen Zeolithen bei 40 bis 100⁰C unter Rühren ca. 24 h aufzuschlämmen. Nach Abfiltrieren, Trocknen bei ca. 150⁰C und Calcinierung bei ca. 500⁰C kann das so gewonnene zeolithische Material mit oder ohne Bindemittel zu Strangen, Pellets oder Wirbelgut weiterverarbeitet werden.

Ein Ionenaustausch des in der H-Form, Ammonium-Form oder Alkali-Form vorliegenden Zeolithen kann so vorgenommen werden, daß man den Zeolithen in Strängen oder Pellets in einer Kolonne vorlegt und darüber z. B. eine wäßrige Ni(NO₃)₂-Lösung oder ammoniakalische Pd(NO₃)₂-Lösung bei leicht erhöhter Temperatur zwischen 30 und 80⁰C im Kreislauf 15 bis 20 h leitet. Danach wird mit Wasser ausgewaschen, bei ca. 150⁰C getrocknet und bei ca. 550⁰C calciniert. Bei manchen metalldotierten Zeolithen, z. B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z. B. so vor, daß man Zeolithe in Pulverform mit 1 n Phosphorsäure 1 Stunde bei 80⁰C behandelt. Nach der Behandlung wird mit Wasser gewaschen, bei 110⁰C/16 Stunden getrocknet und bei 500⁰C/20 Stunden calciniert. Nach einer anderen Arbeitsweise behandelt man Zeolithe vor oder nach ihrer Verformung mit Bindemitteln, z. B. 1 bis 3 Stunden bei Temperaturen von 60 bis 80⁰C mit einer 3 bis 25 gew.%igen, insbesondere 12 bis 20 gew.%igen Salzsäure. Anschließend wird der so behandelte Zeolith mit Wasser gewaschen, getrocknet und bei 400 bis 500⁰C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 bis 2 n, vorzugsweise 0,05 bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z. B. durch Abfiltrieren und Auswaschen, des zeolithischen Materials wird dieses zweckmäßig, z. B. bei Temperaturen von 100 bis 160⁰C, getrocknet und bei Temperaturen von im allgemeinen 450 bis 600⁰C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90⁰C, vorzugsweise 60 bis 80⁰C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z. B. bei Temperaturen von 100 bis 160⁰C, getrocknet und bei Temperaturen von im allgemeinen 450 bis 600⁰C calciniert. Einer HF-Behandlung kann sich auch eine HCl-Behandlung anschließen.

Nach einer anderen Arbeitsweise lassen sich Zeolithe durch Aufbringen von Phosphorverbindungen, wie Trimethylphosphat, Trimethoxiposphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger NaH₂PO₄-Lösung getränkt, bei 110⁰C getrocknet und bei 500⁰C calciniert.

Als acide Heterogenkatalysatoren können auch Schichtsilikate wie Montmorilonit und Bentonit verwendet werden.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphate, Zirkonphosphate, Borphosphate, Eisenphosphate oder deren Gemische.

Als Aluminiumphosphat-Katalysatoren werden für das erfindungsgemäße Verfahren insbesondere unter hydrothermalen Bedingungen synthetisierte Alumiuniumphosphate eingesetzt. Verwendung können z. B. finden AlPO₄, SAPO, MeAPO, MeAPSO, ElAPO und ElAPSO (s. E.M. Flanigen et al. Pure & Appl. Chem. 58 (1986) 1351 ff. ) und Aluminiumphosphate vom MCM-Typ.

Borphosphate für das erfindungsgemäße Verfahren lassen sich beispielsweise durch Mischung und Kneten von konzentrierter Borsäure und durch anschließende Trocknung und Calcination in Inertgas-, Luft- oder Dampfatmosphäre bei 250 bis 650⁰C, vorzugsweise 300 bis 500⁰C herstellen.

Als Phosphat-Katalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x H₂O in 700 ml Wasser über 2 h zugetropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt und ausgewaschen. Er wird bei 60⁰C/16 h getrocknet.

Ein für das erfindungsgemäße Verfahren eingesetztes Cerphosphat wird z. B. durch Fällung aus 52 g Ce(NO₃)₃ x 6 H₂O und 56 g NaH₂PO₄ x 2 H₂O erhalten. Nach der Filtration wird das Material zu Strängen veformt, bei 120⁰C getrocknet und bei 450⁰C calciniert.

Auf diese Phosphate können durch Imprägnierung (Tränken und Aufsprühen) oder in manchen Fällen auch durch Ionenaustausch Modifizierungskomponenten wie voran bei den Zeolithen beschrieben aufgebracht werden. Auch kann wie bei den Zeolithkatalysatoren eine Modifizierung mit Säure, z. B. Phosphorsäure erfolgen.

Ein Phosphorsäure-haltiger Katalysator kann beispielsweise durch Auftränken von Na₃PO₄- oder NaH₂HPO₄- oder Na₂HPO₄-Lösung auf einen Träger wie SiO₂ und anschließende Trocknung bzw. Calcination erhalten werden. Phosphorsäure kann aber auch mit Kieselgel zusammen mit einem Sprühturm versprüht werden, danach schließt sich eine Trocknung und meist eine Calcination an. Phosphorsäure kann auch in einer Imprägniermühle auf das Trägermaterial aufgesprüht werden.

Weiterhin können für das erfindungsgemäße Verfahren vorteilhaft sauer wirkende Oxide, z. B. solche von Elementen der dritten und vierten Hauptgruppe sowie der zweiten bis sechsten Nebengruppe des periodischen Systems, insbesondere Oxide wie Siliciumdioxid in Form von Kieselgel, Kieselgur, Quarz, weiterhin Zinkoxid, Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide verwendet werden. Oxidgemische vorgenannter Oxide sind z. B. Aluminiumoxid wie gamma-Al₂O₃ mit Boroxid, Siliciumdioxid, Wolframoxid oder Chromoxid. Die Oxide können durch Aufbringen von Modifizierungskomponenten wie voranstehend bei den Zeolithkatalysatoren beschrieben dotiert werden. Die Behandlung mit Säuren wie bei den Zeolithkatalysatoren beschrieben ist ebenfalls eine Möglichkeit der Modifizierung.

Die hier beschriebenen Katalysatoren können wahlweise als 2- bis 4-mm-Stränge oder als Tabletten mit 3 bis 5 mm Durchmesser oder als Splitt mit Teilchengrößen von 0,05, insbesondere 0,1 bis 0,5 mm oder als Wirbelkontakt eingesetzt werden. Das Wirbelgut läßt sich z. B. durch Zerkleinern und Aussieben von Strängen oder auch Sprühtrocknung herstellen.

Das vorteilhafte Ergebnis des erfindungsgemäßen Verfahren konnte aufgrund der technischen Kenntnisse nicht erwartet werden. Der Literatur ist vielmehr zu entnehmen, daß Carbonsäureester in der Gasphase in Gegenwart acider Heterogenkatalysatoren unter Abspaltung der Carbonylfunktion in gesättigte und vor allem ungesättigte Kohlenwasserstoffe überführt werden (z. B. EP-A-135 436, US-A-4 102 938, JP 50/47904).

Cyclopentenone stellen wertvolle Zwischenprodukte für die Synthese von Duftstoffen und Naturstoffen, u.a. Prostaglandinen, dar. Das α,β-ungesättigte Ketonsystem in den 2-Cyclopentenonen ermöglicht eine Vielzahl von Additionsreaktionen vom Michael- oder Diels-Alder-Typ.

### Beispiel 1

### Herstellung des Katalysators:

Ein Aluminosilikatzeolith vom Pentasil-Typ wurde unter hydrothermalen Bedingungen bei autogenem Druck und 150⁰C aus 650 g hochdispersem SiO₂, 203 g Al₂(SO₄)₃ x 18 H₂O in 10 kg einer wäßrigen 1,6-Diaminohexan-Lösung (Mischung 50 : 50 Gew.-%) in einem Rührautoklaven hergestellt. Nach Abfiltrieren und Auswaschen wurde das kristalline Reaktionsprodukt bei 110⁰C/24 h getrocknet und bei 500⁰C/24 h calciniert. Dieser Aluminosilikatzeolith enthielt 92,8 Gew.-% SiO₂ und 4,2 Gew.-% Al₂O₃.

Zur Herstellung des Katalysators wurde der Alusilikatzeolith mit HF behandelt. Hierbei wurden 50 g des Alusilikatzeolithen mit einer Mischung aus 140 ml 0,1 n HF und 40 ml Wasser unter Rückfluß gekocht. Nach Abfiltrieren wurde mit Wasser neutral gewaschen, bei 110⁰C/16 h getrocknet und bei 500⁰C/5 h calciniert. Dieses Material wurde mit amorphem Aluminosilikat (SiO₂ : Al₂O₃ = 75 : 25 Gew.-%) im Massenverhältnis 60 : 40 verstrangt. Danach wurde bei 110⁰C/16 h getrocknet und bei 500⁰C/16 h calciniert.

### Herstellung von 2-Cyclopenten-1-on aus 3-Pentensäuremethylester:

49,8 g 3-Pentensäuremethylester (cis/trans-Gemisch) wurden pro Stunde bei Atmosphärendruck verdampft und bei 450⁰C über obigen Katalysator (Schüttdichte 0,52 kg pro Liter, Reaktorinnendurchmesser 25 mm) geleitet. Die Katalysatorbelastung betrug 0,50 kg Ester pro Liter Katalysator und Stunde und die Inertgasbelastung 50 l Stickstoff pro Liter Katalysator und Stunde. Die entstandenen Reaktionsdämpfe kondensierte man. Nach 2 Stunden wurden 86,6 g Reaktionsgemisch erhalten, welches gemäß quant. Gaschromatographie 13,1 Gew.-% 2-Pentensäuremethylester, 51,8 Gew.-% 3-Pentensäuremethylester, 3,6 Gew.-% 4-Pentensäuremethylester und 14,1 Gew.-% Cyclopentenon enthielt. Dies entspricht einem Gesamt-Pentensäuremethylester-Umsatz von 40,4 % und einer 2-Cyclopentenon-Selektivität von 42,2 %.

### Beispiel 2

### Herstellung des Katalysators:

Der Borozeolith des Pentasiltyps wurde in einer hydrothermalen Synthese aus 640 g hochdispersen SiO₂, 122 g H₃BO₃, 8 000 g einer 50 %ig wäßrigen 1,6-Diaminohexan-Lösung bei 170⁰C unter autogenem Druck in einem Rührautoklaven hergestellt. Nach Abfiltrieren und Auswaschen wurde das kristalline Reaktionspodukt 24 Stunden bei 100⁰C getrocknet und 24 Stunden bei 500⁰C calciniert. Dieser Borosilikatzeolith enthält 94,2 Gew-% SiO₂ und 2,3 Gew.-% B₂O₃.

Mit diesem Material wurden durch Verformen mit einem Verformungshilfsmittel 2-mm-Stränge hergestellt, die bei 110⁰C 16 Stunden getrocknet und bei 500⁰C 24 Stunden calciniert wurden.

### Herstellung von 2-Cyclopenten-1-on aus 3-Pentensäuremethylester:

32,5 g 3-Pentensäuremethylster wurden pro Stunde bei Atmosphäredruck verdampft und bei 450⁰C über den Borosilikatzeolithen (Schüttdichte 0,37 kg, Reaktorinnendurchmesser 20 mm) geleitet. Bei einer Katalysatorbelastung von 0,50 kg Ester pro Liter Katalysator und Stunde sowie einer Inertgasbelastung von 77 l Stickstoff pro Liter Katalysator und Stunde fielen nach 2 Stunden 52,8 g eines Produktgemisches, das gemäß quant. Gaschromatographie 6,2 Gew.-% 2-Pentensäuremethylester, 10,0 Gew.-% 3-Pentensäuremethylester, 3,5 Gew.-% 4-Pentensäuremethylester und 34,0 Gew.-% 2-Cyclopentenon enthielt. Dies entspricht einer Cyclopentenon-Selektivität von 45,7 % bei einem Gesamt-Pentensäuremethylester-Umsatz von 84,0 %

### Beispiel 3

### Herstellung des Katalysators:

Der cäsiumhaltige Borosilikatzeolith wurde erhalten, indem man die Stränge des Borosilikatzeolithen aus Beispiel 2 mit einer wäßrigen Cs₂CO₃-Lösung imprägniert, danach 2 Stunden bei 130⁰C trocknet und 2 Stunden bei 540⁰C calciniert. Der Cs-Gehalt beträgt 0,6 Gew.-%.

### Herstellung von 2-Cyclopenten-1-on aus 3-Pentensäuremethylester:

3-Pentensäuremethylester wurden bei 255⁰C unter Atmosphärendruck verdampft und bei 450⁰C über 100 ml des Cäsium-dotierten Borosilicatzeolithen (Schüttdichte 0,40 kg pro Liter, Reaktorinnendurchmesser 25 mm) geleitet. Bei einer Katalysatorbelastung von 0,51 kg Ester pro Liter Katalysator und Stunde sowie einer Inertgasbelastung von 100 l Stickstoff pro Liter Katalysator und Stunde erhielt man durch Kondensation nach 5 Stunden 199,6 g Produktgemisch folgender Zusammensetzung: 6,6 Gew.-% 2-Pentensäuremethylester, 10,1 Gew.% 3-Pentensäuremethylester, 3,4 Gew.-% 4-Pentensäuremethylester und 36,8 Gew.% 2-Cyclopentenon, entsprechend einer Cyclopentenonselektivität von 47,4 % bei einem Gesamt-Pentensäureester-Umsatz von 84,3 %.

### Beispiel 4

### Herstellung von 2-Cyclopenten-1-on aus 2-Pentensäuremethylester:

In einem Quarzrohr verdampfte man bei Atmosphärendruck pro Stunde 50,0 g 2-trans-Pentensäuremethylester und leitet die Eduktdämpfe gemeinsam mit 10 l Stickstoff bei 450⁰C über 100 ml eines Cäsium-dotierten Borosilikatzeolithen aus Beispiel 3 (Schüttdichte 0,40 kg pro Liter, Reaktorinnendurchmesser 25 mm); die Reaktionsdämpfe wurden kondensiert. Nach 4 Stunden erhielt man 158,7 g Reaktionsgemisch, das sich folgendermaßen zusammensetzte: 0,6 Gew.-% 2-Pentensäuremethylester, 1,2 Gew.-% 3-Pentensäuremethylester, 1,3 Gew.-% 4-Pentensäuremethylester und 39,8 Gew.-% Cyclopentenon; dies entspricht einem Gesamt-Pentensäuremethylester-Umsatz von 97,5 % und einer 2-Cyclopentenon-Selektivität von 45,1 %.

### Beispiel 5

### Herstellung von 2-Cyclopentenon aus 4-Pentensäuremethylester:

Aus einem Verdampfer wurden bei Normaldruck pro Stunde 47,2 g 4-Pentensäuremethylester mit 10 l Stickstoff in einen auf 450⁰C beheizten Reaktor (Reaktorinnendurchmesser 25 mm) eingeleitet, der mit 100 ml des Cäsium-dotierten Borosilicatzeolithen aus Beispiel 3 gefüllt war. Durch Kondensation der Reaktionsdämpfe erhielt man nach 2-stündigem Betrieb 88,8 g Reaktionsgemisch der folgenden Zusammensetzung (quant. GC): 6,4 Gew.-% 2-Pentensäuremethylester, 11,2 Gew.-% 3-Pentensäuremethylester, 11,4 Gew.-% 4-Pentensäuremethylester und 32,6 Gew.-% 2-Cyclopentenon, was einem Gesamt-Pentensäuremethylester-Umsatz von 72,7 % und einer 2-Cyclopentenon-Selektivität von 58,6 % entspricht.

### Beispiel 6

### Herstellung von 3-Methyl-2-cyclopenten-1-on aus 4-Methyl-4-pentensäuremethylester:

Aus einem Verdampfer wurden bei Normaldruck pro Stunde 22,2 g 4-Methyl-4-hexensäuremethylester mit 5 Liter Stickstoff durch einen auf 350⁰C beheizten Reaktor (Innendurchmesser 25 mm) geleitet, der mit 50 ml des Cäsium-dotierten Borzeolithen aus Beispiel 3 gefüllt war. Die Reaktionsdämpfe kondensierte man. Nach 2,5-stündigem Betrieb wurden 53,7 g Reaktionsgemisch mit 0,6 Gew.-% 4-Methyl-4-pentenester und 55,1 Gew.-% 3-Methyl2-cyclopenten-1-on erhalten, entsprechend einer Selektivität von 71,2 % bei 99,4 % Umsatz.

### Beispiel 7

### Herstellung von 2-Methyl-2-cyclopenten-1-on und 2-Cyclohexen-1-on aus Hexensäuremethylester:

Pro Stunde wurden 27,4 g Hexensäuremethylester (Doppelbindungsisomerengemisch mit 55 % 5-Hexenester und 37 % 4-Hexenester) mit 5 Liter Stickstoff durch einen mit 50 ml des Cäsium-dotierten Borzeolithen aus Beispiel 3 gefüllten Reaktor (Innendurchmesser 25 mm) geleitet. Kondensation der Reaktionsdämpfe lieferte nach 4 Stunden 105,1 g Reaktionsgemisch, in dem gemäß quant. Gaschromatographie 8,8 g Hexensäuremethylester (Isomerengemisch), 25,2 g 2-Methyl-2-cyclopenten-1-on und 28,8 g 2-Cyclohexen-1-on enthalten waren. Dies entspricht einem Hexenester-Umsatz von 92,0 % sowie Selektivitäten von 33,3 % für 2-Methylcyclopentenon und 38,1 % für Cyclohexenon.

### Beispiel 8

### Herstellung von 2-Methyl-2-cyclopenten-1-on und 2-Cyclohexen-1-on aus Hexensäuremethylester:

Aus einem Verdampfer wurden bei Normaldruck pro Stunde 48,9 g Hexensäuremethylester (Doppelbindungsisomerengemisch mit 55 % 5-Hexenester und 37 % 4-Hexenester) mit 10 l Stickstoff in einen auf 450⁰C beheizten Reaktor (Reaktorinnendurchmesser 25 mm) eingeleitet, der mit 100 ml des Cäsiumdotierten Borosilicatzeolithen aus Beispiel 3 gefüllt war. Durch Kondensation der Reaktionsdämpfe erhielt man nach 3-stündigem Betrieb 139,0 g Reaktionsgemisch der folgenden Zusammensetzung (quant. GC): 1,3 Gew.-% Hexenester (Isomerengemisch) 29,4 Gew.-% 2-Methyl-2-cyclopenten-1-on und 19,4 Gew.-% 2-Cyclohexen-1-on, entsprechend einem Umsatz von 98,8 % und Selektivitäten von 37,5 % für 2-Methylcyclopentenon und 24,8 % für Cyclohexenon.

## Patentansprüche

1. Verfahren zur Herstellung von Cyclopentenonen der allgemeinen Formel I 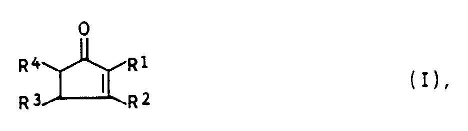 in der R¹ bis R⁴ unabhängig voneinander Wasserstoff oder C₁- bis C₈-Alkyl bedeuten, dadurch gekennzeichnet, daß man Carbonsäureester der allgemeinen Formeln IIa und/oder IIb und/oder IIc
R¹CH=CR²-CHR³-CHR⁴-CO₂R⁵ (IIa)
und/oder
R¹CH₂-CR²=CR³-CHR⁴-CO₂R⁵ (IIb)
und/oder
R¹CH₂-CHR²-CR³=CR⁴-CO₂R⁵ (IIc),
in der R¹ bis R⁴ die obengenannten Bedeutungen haben und R⁵ für C₁- bis C₈-Alkyl steht, an aciden Heterogenkatalysatoren bei Temperaturen von 50 bis 800°C und Drücken von 0,001 bis 50 bar umsetzt.

2. Verfahren nach Anspruch 1, dadurch gekennzeichnet, daß die Substituenten R¹ bis R⁴ unabhängig voneinander Wasserstoff oder C₁- bis C₄-Alkyl bedeuten.

3. Verfahren nach Anspruch 1, dadurch gekennzeichnet, daß die Substituenten R¹ bis R⁴ unabhängig voneinander Wasserstoff oder Methyl bedeuten.

4. Verfahren nach Anspruch 1, dadurch gekennzeichnet, daß der Substituent R⁵ in Formel II C₁- bis C₄-Alkyl bedeutet.

5. Verfahren nach Anspruch 1, dadurch gekennzeichnet, daß der Substituent R⁵ in Formel II Methyl bedeutet.

6. Verfahren nach Anspruch 1, dadurch gekennzeichnet, daß man die Umsetzung in der Gasphase bei Temperaturen von 150 bis 800°C und Drücken von 0,001 bis 50 bar durchführt.

7. Verfahren nach Anspruch 1, dadurch gekennzeichnet, daß man die Umsetzung an sauren Zeolithen, Phosphaten und/oder sauer wirkenden Oxiden der Elemente der 3. und 4. Hauptgruppe sowie der 2. bis 6. Nebengruppe des periodischen Systems vornimmt.

8. Verfahren nach Anspruch 1, dadurch gekennzeichnet, daß man die Umsetzung an sauren zeolithischen Katalysatoren vornimmt.

## Claims

1. A process for preparing a cyclopentenone of the formula I 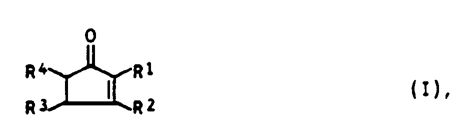 where each of R¹ to R⁴ is independently of the others hydrogen or C₁-C₈-alkyl , comprising converting a carboxylic ester of the formula IIa and/or IIb and/or IIc
R¹CH=CR²-CHR³-CHR⁴-CO₂R⁵ (IIa)
and/or
R¹CH₂-CR²=CR³-CHR⁴-CO₂R⁵ (IIb)
and/or
R¹CH₂-CHR²-CR³=CR⁴- CO₂R⁵ (IIc),
where each of R¹ to R⁴ is as defined above and R⁵ is C₁-C₈-alkyl, over an acidic heterogeneous catalyst at 50 - 800°C and 0.001 - 50 bar.

2. A process as claimed in claim 1, wherein the substituents R¹ to R⁴ are each independently of the others hydrogen or C₁-C₄-alkyl.

3. A process as claimed in claim 1, wherein the substituents R¹ to R⁴ are each independently of the others hydrogen or methyl.

4. A process as claimed in claim 1, wherein the substituent R⁵ in the formula II is C₁-C₄-alkyl.

5. A process as claimed in claim 1, wherein the substituent R⁵ in the formula II is methyl.

6. A process as claimed in claim 1, wherein the reaction is carried out in the gas phase at 150 - 800°C and 0.001 - 50 bar.

7. A process as claimed in claim 1, wherein the reaction is carried out over acidic zeolites, phosphates and/or oxides of the elements of main group 3 or 4 or subgroup 2, 3, 4, 5 or 6 of the periodic table.

8. A process as claimed in claim 1, wherein the reaction is carried out over an acidic zeolitic catalyst.

## Revendications

1. Procédé de fabrication de cyclopenténones de la formule générale I 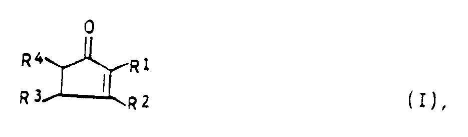 dans laquelle les symboles R¹ à R⁴ représentent, indépendamment les uns des autres, des atomes d'hydrogène ou des radicaux alkyle en C₁ à C₈, caractérisé en ce que l'on fait réagir des esters d'acides carboxyliques des formules générales IIa et/ou IIb et/ou IIc
R¹CH=CR²-CHR³-CHR⁴-CO₂R⁵ (IIa)
et/ou
R¹CH₂-CR²=CR³-CHR⁴-CO₂R⁵ (IIb)
et/ou
R¹CH₂-CHR²-CR³=CR⁴-CO₂R⁵ (IIc),
dans lesquelles les symboles R¹ à R⁴ possèdent les significations qui leur ont été attribuées ci-dessus et R⁵, représente un radical alkyle en C₁ à C₈, sur des catalyseurs hétérogènes et acides, à des températures de 50 à 800°C et sous des pressions de 0,001 à 50 bars.

2. Procédé suivant la revendication 1, caractérisé en ce que les symboles R¹ à R⁴ représentent, indépendamment les uns des autres, des atomes d'hydrogène ou des radicaux alkyle en C₁ à C₄.

3. Procédé suivant la revendication 1, caractérisé en ce que les symboles R¹ à R⁴ représentent, indépendamment les uns des autres, des atomes d'hydrogène ou des radicaux méthyle.

4. Procédé suivant la revendication 1, caractérisé en ce que le symbole R5 représente un radical alkyle en C₁ à C₄ dans la formule II.

5. Procédé suivant la revendication 1, caractérisé en ce que le symbole R5 représente le radical méthyle dans la formule II.

6. Procédé suivant la revendication 1, caractérisé en ce que l'on entreprend la réaction en phase gazeuse, à des températures de 150 à 800°C et sous des pressions de 0,001 à 50 bars.

7. Procédé suivant la revendication 1, caractérisé en ce que l'on entreprend la réaction sur des phosphates, des zéolites acides et/ou des oxydes à activité acide des éléments des troisième et quatrième groupes principaux, ainsi que des second à sixième groupes principaux du système périodique.

8. Procédé suivant la revendication 1, caractérisé en ce que l'on entreprend la réaction sur des catalyseurs zéolitiques acides.
